# EUROPEAN PATENT APPLICATION

(11) **EP 4 748 337 A1**
(43) Date of publication of application: **27.05.2026**
(21) Application number: 25217220.0
(22) Date of filing: 20.11.2025
(51) Int. Cl.: A61B 34/30, A61B 34/37

(54) **VISUALIZATION OF EFFECTS OF DEVICE PLACEMENT IN AN OPERATING ROOM**

(30) Priority: 20.11.2024 US 202418954205
(71) Applicant: Cilag GmbH International, 6300 Zug (CH)
(72) Inventor: SHELTON IV,, Frederick E., Cincinnati, 45242 (US); BRADY,, John E., Cincinnati, 45242 (US); JONES,, Shannon L., Cincinnati, 45242 (US); HARRIS,, Jason L., Cincinnati, 45242 (US); ADAMS,, Shane R., Cincinnati, 45242 (US); DECK,, Andrew C., Cincinnati, 45242 (US); GRABOWSKI,, Cole, Cincinnati, 45242 (US); RUSHTON,, Dylan, Cincinnati, 45242 (US); MUMAW,, Daniel James, Cincinnati, 45242 (US); KAMINSKI,, Benjamin M., Cincinnati, 45242 (US); ARONHALT,, Jacqueline Corrigan, Cincinnati, 45242 (US); JAYME,, Madeleine, Cincinnati, 45242 (US)
(74) Representative: Carpmaels & Ransford LLP

(57) **Abstract**

Devices and methods for visualizing effects of device placement in an operating room. An example device may determine a fixed position of a first base attached to a first robotic arm. The device may determine based on the fixed position of the first base, that a first candidate position of a second base is associated with a first number of interactions in which the first robotic arm and a second robotic arm attached to the second base will co-occupy space. The device may determine, based on the fixed position of the first base, that a second candidate position of the second base is associated with a second number of interactions in which the first robotic arm and the second robotic arm will co-occupy space. The device may select the first candidate position or the second candidate position based on the first number of interactions and the second number of interactions.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November 20, 2024, entitled METHOD FOR MULTI-SYSTEM INTERACTION,
- U.S. Patent Application No. 18/954,195, filed November 20, 2024, entitled, VISUALIZATION OF AN INTERNAL PROCESS OF AN AUTOMATED OPERATION,
- U.S. Patent Application No. 18/954,199, filed November 20, 2024, entitled VISUALIZATION OF AUTOMATED SURGICAL SYSTEM DECISIONS,
- U.S. Patent Application No. 18/954,178, filed November 20, 2024, entitled VISUALIZATION OF EFFECTS OF DEVICE MOVEMENTS IN AN OPERATING ROOM, and
- U.S. Patent Application No. 18/954,214, filed November 20, 2024, entitled DISPLAY OF COMPLEX AND CONFLICTING INTERRELATED DATA STREAMS.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Devices and methods for visualizing the effect of device placement in an operating room. An example device may include a processor configured to perform one or more actions. The device may receive an indication of a plurality of steps of a surgical procedure. One or more steps in the plurality of steps of the surgical procedure involve use of at least one of a first robotic arm attached to a first base, or a second robotic arm attached to a second base. The device may determine a fixed position of the first base. The device may determine, based on the plurality of steps of the surgical procedure and the fixed position of the first base, that a first candidate position of the second base is associated with a first number of interactions in which the first robotic arm and the second robotic arm will co-occupy space during the surgical procedure. The device may determine, based on the plurality of steps of the surgical procedure and the fixed position of the first base, that a second candidate position of the second base is associated with a second number of interactions in which the first robotic arm and the second robotic arm will co-occupy space during the surgical procedure. The device may select a candidate position for the second base, from the first candidate position and the second candidate position, based on the first number of interactions and the second number of interactions. The device may generate a control signal configured to indicate the selected candidate position for the second base.

The control signal being configured to indicate the selected candidate position of the second base comprises the control signal being configured to indicate one or more of: the first candidate position, the first number of interactions, the second candidate position, the second number of interactions, and a recommendation for the selected candidate position to be used as a fixed position of the second base.

The first robotic arm may be configured to move a first end effector attached to a distal end of the first robotic arm, and the second robotic arm is configured to move a second end effector attached to a distal end of the second robotic arm, each step in the plurality of steps of the surgical procedure is associated with a surgical space internal to a patient. The device may identify a set of candidate positions, comprising the first candidate position and the second candidate position, based on the plurality of steps of the surgical procedure. Each candidate position in the set of candidate positions may allow the first end effector and the second end effector to access the surgical space at a given step in the plurality of steps of the surgical procedure.

On a condition that the first number of interactions is less than the second number of interactions, the device may select the first candidate position. On a condition that the first number of interactions is greater than the second number of interactions, the device may select the second candidate position.

One or more steps in the plurality of steps of the surgical procedure may involve use of a third robotic arm attached to a third base. The device may determine, based on the plurality of steps of the surgical procedure, the fixed position of the first base, and the selected candidate position, that a third candidate position of the third base is associated with a third number of interactions in which the third robotic arm and at least one of the first robotic arm or the second robotic arm will co-occupy space during the surgical procedure. The device may determine, based on the plurality of steps of the surgical procedure the fixed position of the first base, and the selected candidate position, that a fourth candidate position of the third base is associated with a fourth number of interactions in which the third robotic arm and at least one of the first robotic arm or the second robotic arm will co-occupy space during the surgical procedure. The device may select a candidate position for the third base, from the third candidate position and the fourth candidate position, based on the third number of interactions and the fourth number of interactions. The device may generate a control signal configured to indicate the selected candidate position for the third base.

One or more steps in the plurality of steps of the surgical procedure may involve use of a third robotic arm attached to a third base. The device may predict an effect, caused by the selected candidate position for the second base, on placement of the third base, wherein the control signal is further configured to indicate the effect.

The device may receive user preference information. The device may determine a surgical constraint based on the user preference information. The device may select the candidate position for the second base, from the first candidate position and the second candidate position, based on the surgical constraint.
The device may determine a patient position associated with the surgical procedure. The device may determine a surgical constraint based on the patient's position. The device may select the candidate position for the second base, from the first candidate position and the second candidate position, based on the surgical constraint.

### BRIEF DESCRIPTION OF THE DRAWINGS

FIG. 1 is a block diagram of a computer-implemented surgical system.
FIG. 2 illustrates an example surgical system in a surgical operating room.
FIG. 3 illustrates an example surgical hub paired with various systems.
FIG. 4 illustrates an example situationally aware surgical system.
FIG. 5 illustrates an example robotic arm attached to a base.
FIG. 6 illustrates an example operating room arrangement of multiple robotic arms.
FIG. 7 illustrates an example display to a user of the effects of device placement in an operating room.
FIG. 8 illustrates an example flow chart for device placement during operating room set-up initialization.
FIG. 9 illustrates an example flow chart for device placement optimization.
FIG. 10 illustrates an example three-dimensional matrix with the different variables associated with the system, arms, devices, etc.

### DETAILED DESCRIPTION

A more detailed understanding may be had from the following description, given by way of example in conjunction with the accompanying drawings.

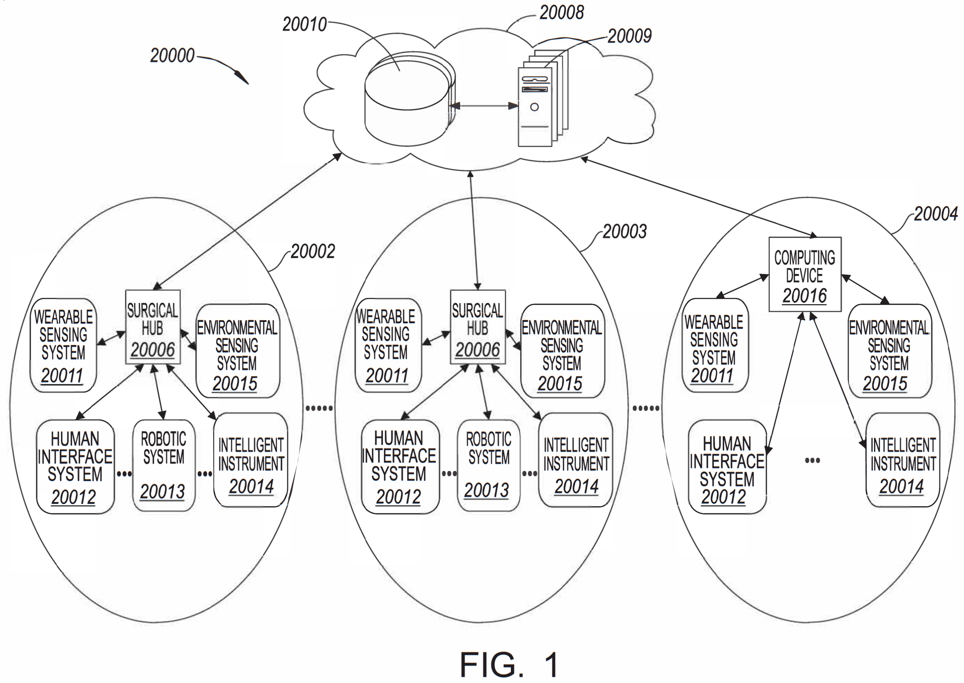

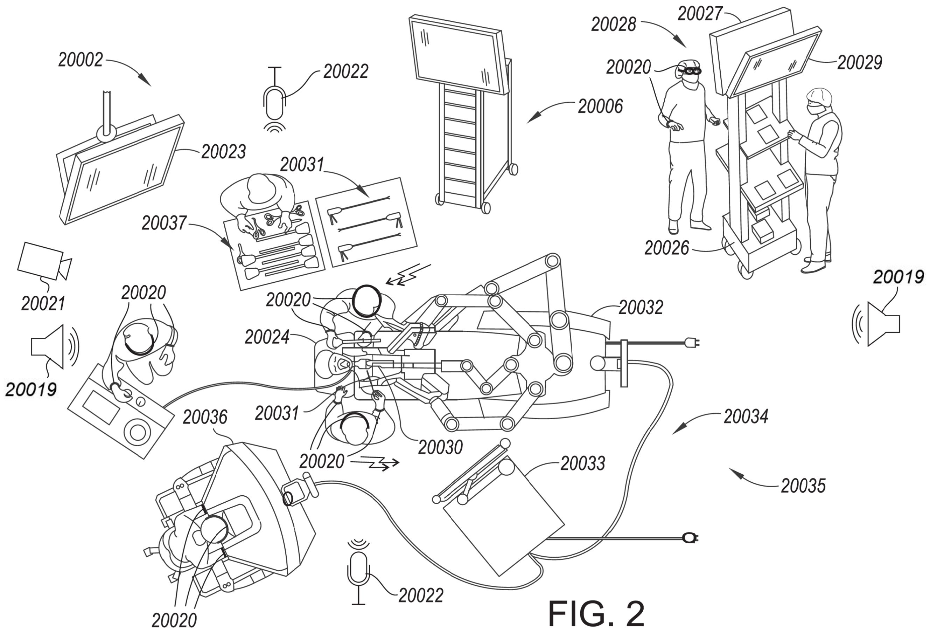

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

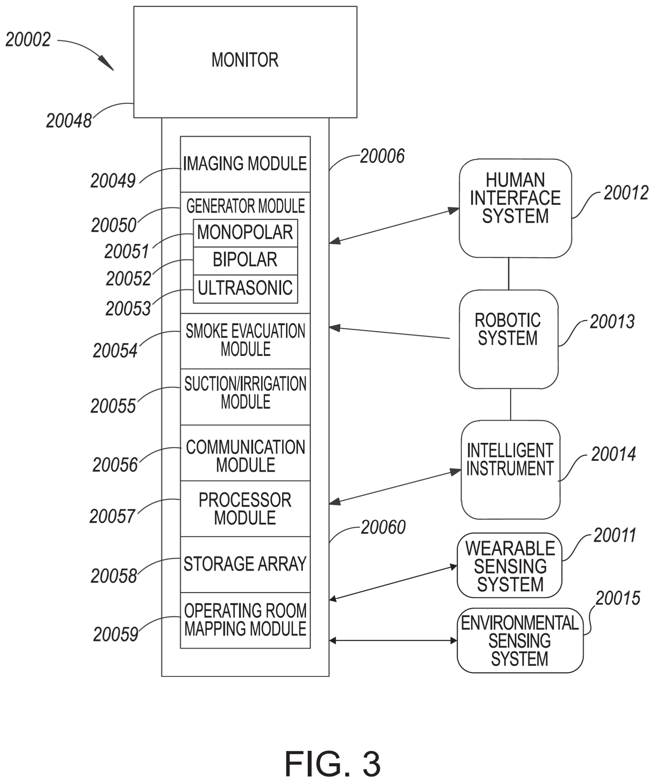

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

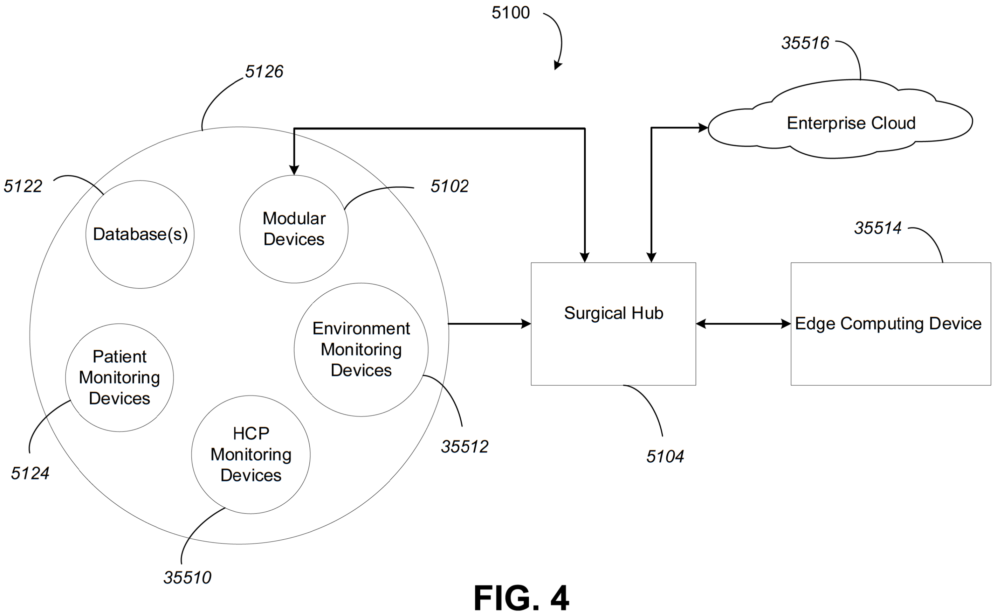

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

Robotic arm placement may be optimized to determine (e.g., and indicate) potential arm interaction(s). The placement may be selected to help mitigate entanglement and/or collisions of the robotic arms.

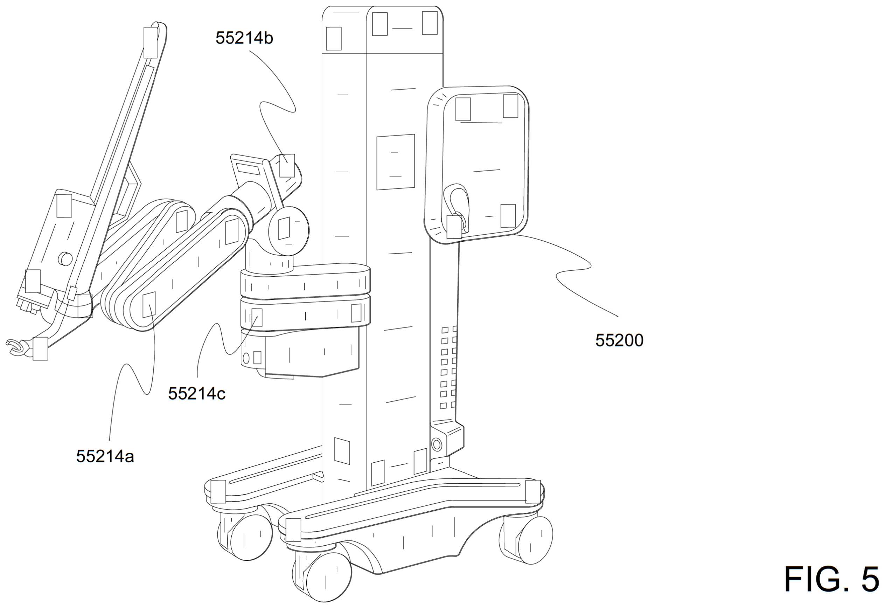

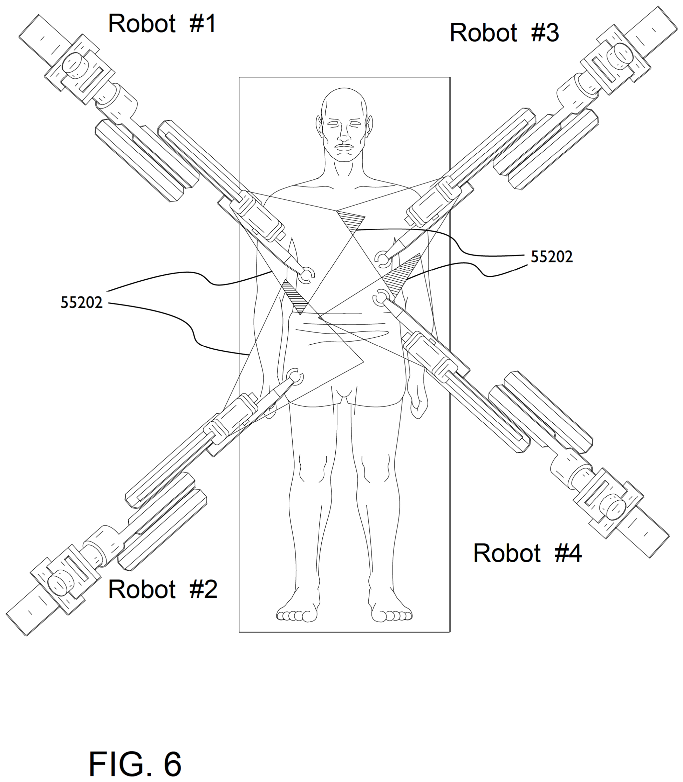

A user (e.g., a health care provider (HCP)) may select the placement of a first surgical instrument (e.g., a base of a robotic arm). FIG. 5 illustrates an example robotic arm 55200 attached to a base. FIG. 6 illustrates an example operating room arrangement of multiple robotic arms. As shown, multiple robotic arms may be present in a relatively small space in an OR. The arms may have ranges of motion that overlap (e.g., as shown at 55202). In this case, the arms may collide unless adjustments are made to reduce the potential interactions. For example, one or more of the arms may not be allowed to occupy the overlapping space (e.g., at a given time). As described herein, strategic placement of the robotic arm bases may help reduce the number of interactions between the arms.

A smart surgical system may determine (e.g., predict) the effect of the placement of the first surgical instrument on the placement of a second surgical instrument. The determination may be based on a predicted progression of a surgical procedure (e.g., where the surgical instruments will be during the steps of the surgical procedure).

The smart system may use the placement of the first surgical device to predict the (e.g., best) placement of the remaining surgical instrument(s). The smart system may display the interaction(s) between the surgical instruments based on the placements. The smart system may display the location(s) of the interaction(s) relative to the surgical site. The smart system may display placement options to the user to allow the user to place or indicate a selected placement location. The smart system may display placement option(s) of other surgical instruments based on the selected location.

The smart system may predict the (e.g., best) placement(s) of the surgical instrument(s) based on the interactions and procedure constraints. The procedure constraints may include the procedure steps, the instruments selected, and the user access specifications (e.g., requirements). The smart system may display placement options to the user. The display may include multiple cascaded placement options. The placement options may be determined based on user prioritization or choices, the layout of the operating room, equipment type/placement, and/or patient positioning. The placement of robotic arm(s) may be determined so that the arm(s) are able to reach the surgical space (e.g., all of the surgical space) inside the patient.

Placement preferences (e.g., from the HCP) may influence criteria for placement determination. Different HCPs (e.g., surgeons) may have varying skillsets, tool preferences, and/or surgical approaches. HCPs may have different approaches to the same surgical procedure. For example, a resident surgeon being overseen by an attending surgeon may use a different surgical approach than an experienced surgeon (e.g., who has performed a large volume of procedures). The criteria for placement determination may change from one HCP to the next.

The system may choose to select device location to allow the device to access the (e.g., all of the) surgical space. The system may select the device location based on one or more constraints (e.g., toolset length, tools to be used, device articulation capabilities, and/or a number of instruments in use, which may be limited due to cavity space, for example). If the device(s) are able to be moved (e.g., manipulated) throughout the procedure, one or more constraint(s) may be flexible.

The device may receive user preference information. The device may determine a surgical constraint based on the user preference information. The device may select the candidate position for the second base, from the first candidate position and the second candidate position, based on the surgical constraint.
The device may determine a patient position associated with the surgical procedure. The device may determine a surgical constraint based on the patient's position. The device may select the candidate position for the second base, from the first candidate position and the second candidate position, based on the surgical constraint.

During a surgical procedure, the movement of end effectors attached to robotic arms may cause arm interactions outside of the patient. The arm interactions may be reduced (e.g., minimized) to avoid collisions and/or entanglements (e.g., that could interrupt the procedure or prevent surgical access). The movement of end effectors may similarly cause interactions between the arms and other objects (e.g., people, stationary devices) in the OR.

The first robotic arm may be configured to move a first end effector attached to a distal end of the first robotic arm, and the second robotic arm is configured to move a second end effector attached to a distal end of the second robotic arm, each step in the plurality of steps of the surgical procedure is associated with a surgical space internal to a patient. The device may identify a set of candidate positions, comprising the first candidate position and the second candidate position, based on the plurality of steps of the surgical procedure. Each candidate position in the set of candidate positions may allow the first end effector and the second end effector to access the surgical space at a given step in the plurality of steps of the surgical procedure.

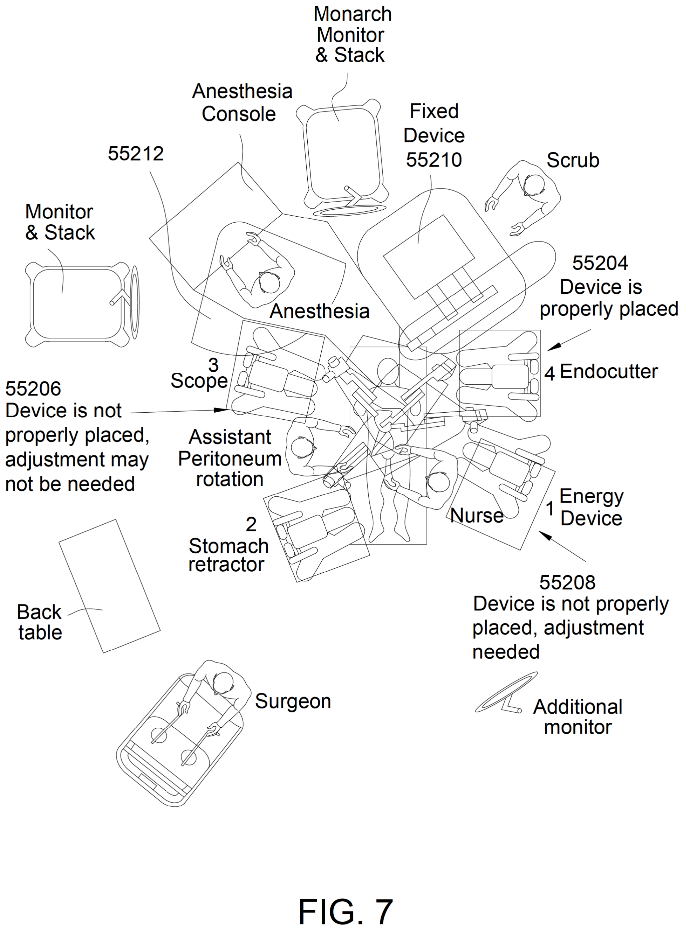

The smart system may predict placement(s) for one or more robotic arms (e.g., the Hugo robot arm). The system may display a recommended placement to the OR team. FIG. 7 illustrates an example display to a user of the effects of device placement in an operating room. For example, as shown at 55204, the system may indicate that a device is properly placed. The system may indicate (e.g., at 55206) that a device is not properly placed, but that the placement is close enough to the recommended placement that adjustment may not be needed. As another example, the system may indicate (e.g., at 55208) that a device is not properly placed and should be adjusted (e.g., because the current placement of the device may cause negative interactions between devices).

FIG. 7 further illustrates an example of a fixed (e.g., non-moving) device 55210. The fixed device 55210 may be a ventilator (e.g., a Monarch smart ventilator). The ventilator may be placed (e.g., and fixed) at the patient's head (e.g., because the ventilator must be attached at the patient's mouth and/or nose). FIG. 7 further illustrates an example area 55212 in which an HCP (e.g., the anesthesiologist) will occupy during one or more parts of the surgery. For example, throughout a surgery, the anesthesiologist may need to have access to the patient's head to properly check and maintain sedation.

Visualization of the placement(s) may differ based on a level of interest that the HCP has in placement prediction. For example, a surgeon performing a procedure that they perform often (e.g., 100 times per month) may want less recommendation for placement (e.g., have less interest in placement prediction). In another example, a surgeon performing a procedure for the first time may prefer heavy intervention (e.g., have a great interest in placement prediction). Intermediate levels of interest may similarly be available. The level of interest may determine an amount of information and/or recommendations displayed to the HCP.

Devices and methods for visualizing the effects of device placement in an operating room. An example device may include a processor configured to perform one or more actions. The device may receive an indication of a plurality of steps of a surgical procedure. One or more steps in the plurality of steps of the surgical procedure involve use of at least one of a first robotic arm attached to a first base, or a second robotic arm attached to a second base. The device may determine a fixed position of the first base. The device may determine, based on the plurality of steps of the surgical procedure and the fixed position of the first base, that a first candidate position of the second base is associated with a first number of interactions in which the first robotic arm and the second robotic arm will co-occupy space during the surgical procedure. The device may determine, based on the plurality of steps of the surgical procedure and the fixed position of the first base, that a second candidate position of the second base is associated with a second number of interactions in which the first robotic arm and the second robotic arm will co-occupy space during the surgical procedure. The device may select a candidate position for the second base, from the first candidate position and the second candidate position, based on the first number of interactions and the second number of interactions. The device may generate a control signal configured to indicate the selected candidate position for the second base.

On a condition that the first number of interactions is less than the second number of interactions, the device may select the first candidate position. On a condition that the first number of interactions is greater than the second number of interactions, the device may select the second candidate position.

The control signal being configured to indicate the selected candidate position of the second base comprises the control signal being configured to indicate one or more of: the first candidate position, the first number of interactions, the second candidate position, the second number of interactions, and a recommendation for the selected candidate position to be used as a fixed position of the second base.

One or more steps in the plurality of steps of the surgical procedure may involve use of a third robotic arm attached to a third base. The device may determine, based on the plurality of steps of the surgical procedure, the fixed position of the first base, and the selected candidate position, that a third candidate position of the third base is associated with a third number of interactions in which the third robotic arm and at least one of the first robotic arm or the second robotic arm will co-occupy space during the surgical procedure. The device may determine, based on the plurality of steps of the surgical procedure the fixed position of the first base, and the selected candidate position, that a fourth candidate position of the third base is associated with a fourth number of interactions in which the third robotic arm and at least one of the first robotic arm or the second robotic arm will co-occupy space during the surgical procedure. The device may select a candidate position for the third base, from the third candidate position and the fourth candidate position, based on the third number of interactions and the fourth number of interactions. The device may generate a control signal configured to indicate the selected candidate position for the third base. The device may predict an effect, caused by the selected candidate position for the second base, on placement of the third base. The control signal may indicate the effect.

Historic data (e.g., an understanding of issues and/or complicated zones) may be used to identify steps and/or situations most affected by the placement selection.

In an example low intervention mode, the smart system may determine to shift boundary condition(s) based on user experience (e.g., increase space/access if system is in training mode).

The system may display example device placements (e.g., layouts) to the user. The layouts may allow the user to select a (e.g., preferred) set up.

During pre-operative planning, the care team (e.g., nurses, surgeon, other doctors, etc.) may perform one or more of the following. The software may have pre-programmed information from clinical knowledge (e.g., for a given surgical procedure, tumor location, or patient characteristics). The system may determine an (e.g., ideal) arm placement based on the information. The software may reside on a robotic system.

A user interface (UI) may display user input options (e.g., patient weight/height/pre-existing conditions/other characteristics, surgeon handedness, tumor scans or location, procedure type, patient orientation during surgery, areas to be accessed during surgery, bed orientation during surgery, number of people in the OR, room size/setup, etc.). The software may be integrated with hospital electronic health record system(s). This integration may allow the system to pull relevant patient data from a database (e.g., to automatically pre-populate procedure, patient, and/or other information). This may reduce the time and effort used for manual data entry. The software may use the information to output options for the configuration of the devices/arms. The system may display the pros and cons of an (e.g., each) option. The system may display expected conflicts/interactions (e.g., at each step). The software may provide a recommendation (e.g., recommended device placement(s)). The surgeon may select device placement(s). The software may have a pre-defined "training mode." The training mode may be used to recommend device/arm placement if the operating surgeon is a new resident. For example, the training mode may recommend placement(s) to give the surgeon more space or access). The recommended placement(s) may be determined based on HCP input (e.g., clinical advice).

An algorithm may be used to determine recommendations for robotic arm placement in pre-operative planning.

The system may present the surgeon with information or feedback with respect to any non-recommended device placement(s). The feedback may be provided in a graphical format and/or a numerical format. The feedback may be represented in terms of a surgical step, absolute metric, or relative metric.

For example, while positioning the robotic arms before surgery, the system may determine that the current location and orientation of the arms is different from the recommended location/orientation of the arms. The system may display information (e.g., on a surgical screen or tablet), for example, an indication of surgical step(s) that will be affected by the current location/orientation (e.g., if a step will be unable to be completed) and/or a percentage loss in access to the surgical site based on the current location/orientation (e.g., 78% of access compared to 100% access if the recommended placement was used).

The system may display an indication of data sources that are available and/or objects that are in the OR.

The surgical suite floor may have a numbered grid for mapping of device positions. The grid may have six-inch by six- inch squares. The grid may be made with increasing density (e.g., for more accurate placement). The grid may be divided into quadrants (e.g., for basic placement). Software may be used to display a drop down for the HCP to select the surgical procedure being performed and/or available devices. The software may suggest a type and/or number of devices, and/or device placement on the grid. When the devices are placed, an overhead camera may check for placement accuracy (and indicate when the devices are properly placed). During the procedure, if a device moves outside a suggested boundary, the system may provide a warning.

The OR space may be sub-divided into interactive segments. The segments may allow for prioritization or nested movement (e.g., if the OR is reorganized during use).

The system may use a surgical suite grid and algorithm to determine device placement.

A display may show the locations of the connected and non-connected devices in an OR (e.g., to confirm the correct set-up is achieved). The display may have a projector displaying "no access zones" for patient- and/or procedure-specific items. The display may project anticipated staff/personnel locations. If the OR set-up of the equipment is complete, the display may confirm that the connected and non-connected devices are in the correct positions. The display may use a color-coding system to signal if devices are correctly positioned. The display may be used to highlight "no access zones" for a procedure or patient. The display may highlight areas in which personnel are able to be during the procedure. A positioning aid in the OR room may be used to confirm proper device placement.

Robotic arms (and/or other surgical devices) may be positioned (e.g., set up) so as to reduce (e.g., minimize) interactions between with other devices during the procedure.

User inputs may be used to determine (e.g., optimize) robotic arm placement. Factors such as available devices, personnel in the room, patient history, surgeon preference, type of procedure, and/or the like may be inputs to provide the user with a starting point (e.g., to optimize the set-up for additional refinement).

One or more inputs may be used to determine the placement of robotic arms. Device placement and access zones may be determined based on user inputs. The inputs may define "no access zones" (e.g., spaces in which the robotic arms cannot enter).

The inputs may include other devices in the OR (e.g., tables, capital equipment, mayo stands, lighting, etc.). These devices have a defined volume that may be used to calculate available space for the robotic arms. The inputs may include a list of personnel (e.g., nurses, scrub tech, anesthesiologist, surgeons, fellows, etc.) and their anticipated locations in the OR. The inputs may include patient history. For example, high risk factors (e.g., past cardiac events) may cause the system to ensure that the HCP has sufficient access to the chest (e.g., to perform emergency open surgery or to ventilate the patient, etc.). The inputs may include the type of procedure (e.g., and basic patient factors such as size, weight, etc.). For example, in an obese patient, trocars may be higher away from the table, which affects placement of the robotic arms. The inputs may include surgeon preferences (e.g., from previous procedures).

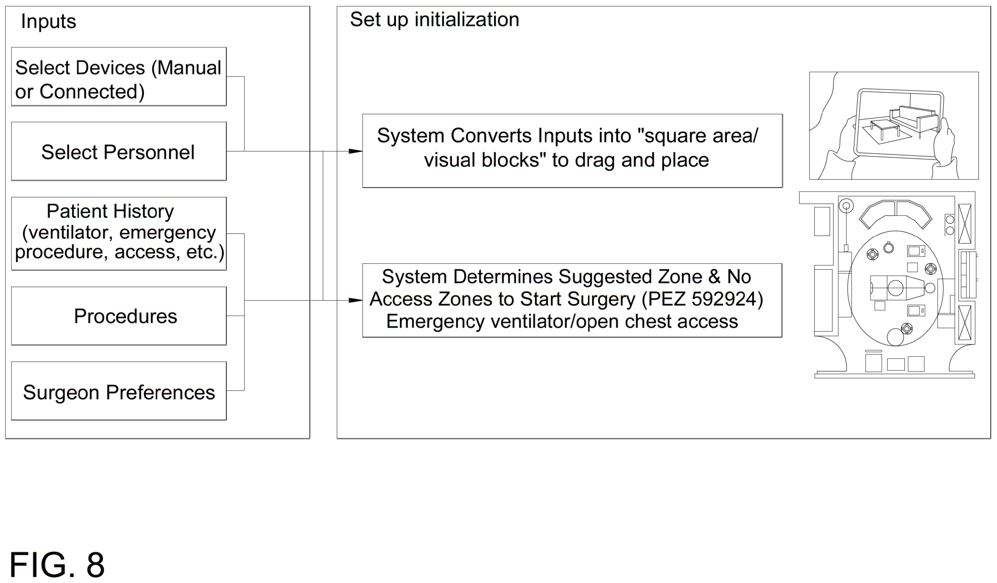

During set-up initialization, a recommended robotic arm placement may be provided to the user. FIG. 8 illustrates an example flow chart for device placement during operating room set-up initialization. As illustrated, the system may receive one or more inputs (e.g., device(s), personnel, patient information, procedure type/steps, surgeon preferences, and/or the like). The system may convert the inputs into a diagram of virtual objects in the OR. The system may indicate recommended device placements and "no access zones." The system may allow a user to alter the initial diagram. For example, the user may drag and place the virtual objects around the diagrammed room. The system may indicate interactions that would result from the user's changes.

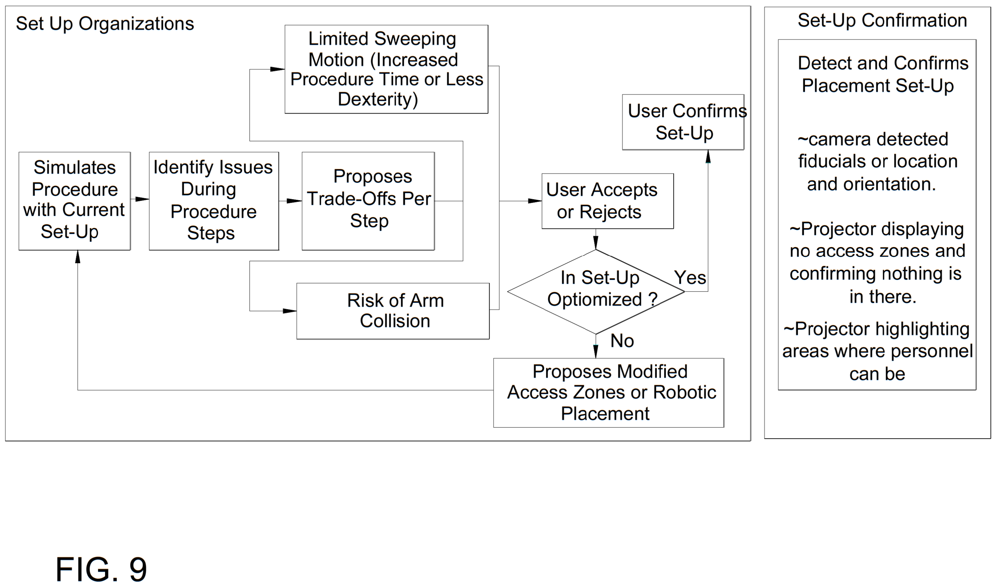

Additional optimization steps may be performed (e.g., after initialization). FIG. 9 illustrates an example flow chart for device placement optimization. As illustrated, the system may simulate the steps of the procedure using the device placements determined during initialization (e.g., including any user adjustments). The system may identify one or more issues/interactions caused by the device placements. The system may indicate trade-offs (e.g., pros and cons) at each step of the procedure. For example, the system may indicate that the device placement will avoid possible interactions at steps 4 and 12, but may create interactions at steps 6 and 19. The system may indicate trade-offs throughout the procedure. For example, the system may indicate that the device placement will reduce the number of interactions by three, but the procedure will take approximately one hour longer than another room setup (e.g., due to the OR staff having to pause and manually rearrange/untangle devices).

The system may indicate options to the user. For example, the system may indicate that limiting the range of motion (e.g., sweeping) of a device would reduce the number of device interactions, but would increase the procedure time or limit the surgeon's ability to access part of the surgical site. The system may indicate the risk of device (e.g., arm) collisions if no preventative action is taken. The system may receive user feedback in response to the options. For example, the user may indicate that they will accept the increased risk of device collisions. In another example, the user may indicate that none of the options are acceptable. In this case, the system may use the feedback to recommend a modified layout of the OR room (e.g., modified device placement) and/or modified access zones for one or more device(s).

If the user selects one of the presented options (e.g., accepts the risk or makes an adjustment), the system may determine that the device placement is finalized. Once the placement is finalized, the system may monitor the device placement and confirm when the devices are correctly positioned. The system may monitor the device placement/orientation by using cameras to track fiducial markers on the devices. The system may display no-access zones in the OR and confirm that no devices are in those zones. The system may also display areas in which the OR staff are able to move (e.g., without coming into contact with surgical device(s)).

Robots may be equipped with means for determining the location of one arm relative to another in three-dimensional space. For example, another may monitor arms and provide relative measurements of one with respect to the other. For example, the system may determine the relative measurements through imaging of the OR. One or more cameras within the OR may be used to generate this information. The cameras may be separate from, or on the robot itself. The system may determine the relative measurements based on magnetic sensors, ultrasonic pinging, etc. This additional data feed may be used to determine the location of the devices relative to each other.

The hub and/or camera system may have to have stored parameter(s) related to the device(s) being tracked and/or capabilities of those device(s). Triangulation of the device position may be used to suggest device motions. For example, the system may use kinematics of the device(s) (e.g., robot arm(s)) and the patient to determine viable movement options. The kinematics may be derived (e.g., on the fly) using visualization. The range of motion (e.g., reach) and balance of the robot may be received from the robot or predetermined.

Indexing elements (e.g., fiducials) on the arms may enable a separate system to monitor the arms, (e.g., each of the segments of the arms). In some examples, electronic sensors may be used (e.g., rather than fiducials). The electronic sensors may emit a signal that is received by other sensors or a base station. For example, fiducials and/or electronic sensors may be placed at points 55214-c (e.g., and/or other joints) in FIG. 5.

An example smart system interaction architecture is provided herein.

The system may detect (and alert the surgical staff of) missing equipment (e.g., before a procedure begins). Cameras in the OR may publish information to a software system (e.g., that is pre-trained with computer vision object detection on an OR dataset (e.g., with all the common tools, equipment, people, etc.). The software may compare the items in the OR to a checklist (pre-)selected by the surgical staff (e.g., before the procedure). The checklist may be determined based on (pre-)programmed information about tools used for a selected procedure type. The information may be displayed to the staff on a computer/monitor screen or indicated in another way (e.g., audio cues, beeps, etc.). The staff may determine (e.g., and indicate via the UI) whether a tool that was indicated as missing is missing or not. That information may be used to improve the algorithm (e.g., in real time).

OR staff may be identified (e.g., by role). For example, the system may identify bedside OR staff and other/additional staff in the room. OR staff may be identified based on an OR dress code (e.g., latex gloves vs. surgical gloves). Bedside staff may wear a different color for a camera to use in identification. Wearables (e.g., wristbands) for staff members may allow the system to track individuals. OR staff may be identified based on the procedure type (e.g., in procedures where the patient is held in a twilight state, anesthetist/anesthesiologist may be more active near the patient and use more space near a ventilation system). OR staff may be identified based on location (e.g., staff in a sterile field may be differentiated from non-sterile staff). OR staff may be identified using a tracking method (e.g., camera-based tracking). OR staff may be identified using visual/image processing (e.g., IR camera, specified markers/wearables, ME field, etc.).

The OR staff members that are tracked may vary over time. For example, not all staff members may be tracked. For example, to prevent robot arm collisions, the system may (e.g., only) track the people in the space the robot is/may be using. A touch point may be included on a robot arm (or elsewhere). The touch point may be used to inform the system that the individual who interacted with the touch point is to be tracked for collision avoidance.

The system may use the procedure plan/type to predict upcoming movements/interactions.

In an example, a ventilator may be stationary (e.g., have no base movement/tubing adjustability without hardware modification). The system may have access to data from the ventilator, but be unable to modify the position/orientation of the ventilator. The system may highlight a larger collision/no fly zone for such stationary equipment (e.g., compared to a moveable device such as a Hugo robot, where the arms can be manipulated). An HCP may prefer to place equipment with limited mobility near areas used for emergency access (e.g., because the equipment cannot move to block the area).

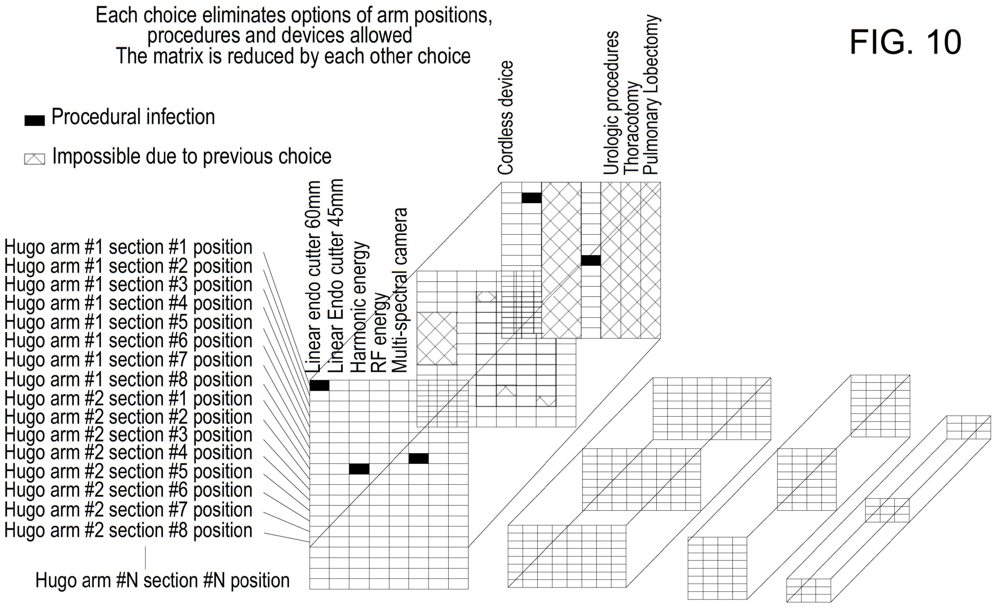

As illustrated in FIG. 10, the system may create a 3D matrix to list the different variables associated with the system, arms, devices, etc. The variable list may include the inputs described herein and/or more specific information (e.g., the parts/joints of a robot arm). For example, a robot arm may have five variables associated with four arm segments and a 360-degree rotatable joint. The variable list may include a variable for the pedestal location of the floor of the OR and/or other joints (e.g., two additional joints, for example, for reach and height). The joint capabilities may determine the pitch, roll, and yaw of the robotic arm system. The variables may be associated with an arm placement (e.g., a single arm placement) within the OR. The number of variables may be multiplied by the number of arm pedestals being used. Additional robotic system consoles, video displays, and/or other capital equipment and devices may become part of the matrix. The number of variables and outcomes/interactions would be improbable (if not impossible) for a human to process. The 3D matrix illustrated in FIG. 10 may whittle down the possible variations.

The surgeon and staff may determine priorities associated with functions and/or parts of surgical equipment (e.g., criticality for a successful outcome). A highest priority device may be placed (e.g., first) and fixed within the OR. The placement may eliminate other equipment from occupying that space. As devices are placed, portions within the matrix may be canceled (e.g., due to conflicts with the already placed devices). For example, if a first robotic arm is placed, the area where the pedestal/base is located and the surrounding arc that the arm will sweep through may not be allowed within the matrix for other equipment. As the OR team begins determining the placement for a second robotic arm, some areas may be excluded within the matrix (e.g., that can be referenced to ensure no unintended interactions take place, for example, the arms running into each other). This process may be repeated as each device is placed and checked off (e.g., until the OR suite is set up).

The OR set-up optimization (e.g., using the matrix) may be sequential (e.g., based on other equipment that has been locked down previously) rather than singular holistic (e.g., presenting all options and proposed locations for all equipment at once). The OR system may select to place an (e.g., only one) item at a time (e.g., rather than try to calculate all possible orientations). For example, the system may (e.g., first) display options for a first robot arm controlling a laparoscope. Once the first robot arm is positioned and locked, the system may prompt the user to place a second robot arm controlling an endocutter (e.g., considering the placement of the first robot arm, and any objects placed at an earlier step or objects that cannot be moved as boundary conditions).

The system may predict placements of devices. The system may display recommended placements to the OR team. The devices may be categorized by movement capability (e.g., a wristed endocutter and a single plane endocutter may have different movement choices). The instruments being used may impact the device placement. The positions of the people in the OR may affect device placement. Steps in which the robot is stopped and steps in which the robot is in use may affect device placement.

Boundary conditions (e.g., including surgical access) may be simulated to improve the likelihood of success of the procedure. An example boundary condition may include the limited articulation of an endocutter. In this case, another device may be used in the endocutter space while the endocutter is not in use.

The system may simulate steps that occur later in the procedure. For example, the system may simulate tumor removal from different port locations.

The system may suggest one or more instruments to use in the procedure. For example, the system may recommend using an ABP device (e.g., instead of an ultrasonic device) due to better robotic access.

The system may use device availability to mitigate future conflicts. For example, the system may suggest using a 45mm endocutter instead of a 60mm endocutter. In another example, the system may suggest using a 45mm energy shaft length instead of a 36mm energy shaft length. The system may recommend using a harmonic (e.g., non-articulating) device or an articulating device (e.g., ENSEAL).

The system may suggest one or more device placements based on a surgeon's use of certain procedure technique(s). For example, if a monopolar tip is used, the system may recommend a distance/proximity between the monopolar tip and a smoke evacuator.

Some devices may affect placement of other devices. For example, robotic arm placement may depend on the location(s) of energy generator(s), laparoscopic monitors, OR tower, lighting, suction/irrigation lines, the position of the patient bed, etc.

Some devices may be initially present and removed later in the procedure. Some devices may be introduced during the surgery and left for the remainder of procedure. A CT machine is an example of a temporarily introduced piece of equipment (e.g., introduced and then removed during the surgery). The decision to introduce or remove devices may be planned pre-procedure and/or determined during the procedure. Capital equipment may refer to devices that are present throughout the procedure.

Sterile and non-sterile equipment may be allowed in different areas.

The system may consider limitations for the humans in the room (e.g., line of sight, for example, if the surgeon wants to be able to see a video screen at any time). The system may determine device-free areas (e.g., based on surgeon selection).

Although some aspects are described with respect to one or more robotic arms, a person of ordinary skill in the art will appreciate that these aspects may be used for any powered device (e.g., an articulable endocutter, etc.).

Example 1. A device comprising:
a processor configured to:
receive an indication of a plurality of steps of a surgical procedure, wherein one or more steps in the plurality of steps of the surgical procedure involve use of at least one of a first robotic arm attached to a first base, or a second robotic arm attached to a second base;
determine a fixed position of the first base;
determine, based on the plurality of steps of the surgical procedure and the fixed position of the first base, that a first candidate position of the second base is associated with a first number of interactions in which the first robotic arm and the second robotic arm will co-occupy space during the surgical procedure;
determine, based on the plurality of steps of the surgical procedure and the fixed position of the first base, that a second candidate position of the second base is associated with a second number of interactions in which the first robotic arm and the second robotic arm will co-occupy space during the surgical procedure;
select a candidate position for the second base, from the first candidate position and the second candidate position, based on the first number of interactions and the second number of interactions; and
generate a control signal configured to indicate the selected candidate position for the second base.

Example 2. The device of example 1, wherein the control signal being configured to indicate the selected candidate position of the second base comprises the control signal being configured to indicate one or more of: the first candidate position, the first number of interactions, the second candidate position, the second number of interactions, and a recommendation for the selected candidate position to be used as a fixed position of the second base.

Example 3. The device of example 1 or 2, wherein the first robotic arm is configured to move a first end effector attached to a distal end of the first robotic arm, and the second robotic arm is configured to move a second end effector attached to a distal end of the second robotic arm, each step in the plurality of steps of the surgical procedure is associated with a surgical space internal to a patient, and wherein the processor is further configured to identify a set of candidate positions, comprising the first candidate position and the second candidate position, based on the plurality of steps of the surgical procedure, wherein each candidate position in the set of candidate positions allows the first end effector and the second end effector to access the surgical space at a given step in the plurality of steps of the surgical procedure.

Example 4. The device of any one of examples 1-3, wherein the processor being configured to select the candidate position, from the first candidate position and the second candidate position, based on the first number of interactions and the second number of interactions comprises the processor being configured to:
on a condition that the first number of interactions is less than the second number of interactions, select the first candidate position; and
on a condition that the first number of interactions is greater than the second number of interactions, select the second candidate position.

Example 5. The device of any one of examples 1-4, wherein one or more steps in the plurality of steps of the surgical procedure involve use of a third robotic arm attached to a third base, and the processor is further configured to:
determine, based on the plurality of steps of the surgical procedure, the fixed position of the first base, and the selected candidate position, that a third candidate position of the third base is associated with a third number of interactions in which the third robotic arm and at least one of the first robotic arm or the second robotic arm will co-occupy space during the surgical procedure;
determine, based on the plurality of steps of the surgical procedure the fixed position of the first base, and the selected candidate position, that a fourth candidate position of the third base is associated with a fourth number of interactions in which the third robotic arm and at least one of the first robotic arm or the second robotic arm will co-occupy space during the surgical procedure;
select a candidate position for the third base, from the third candidate position and the fourth candidate position, based on the third number of interactions and the fourth number of interactions; and
generate a control signal configured to indicate the selected candidate position for the third base.

Example 6. The device of any one of examples 1-5, wherein one or more steps in the plurality of steps of the surgical procedure involve use of a third robotic arm attached to a third base, wherein the processor is further configured to predict an effect, caused by the selected candidate position for the second base, on placement of the third base, wherein the control signal is further configured to indicate the effect.

Example 7. The device of any one of examples 1-6, wherein the processor is further configured to:
receive user preference information; and
determine a surgical constraint based on the user preference information, wherein the processor being configured to select the candidate position for the second base, from the first candidate position and the second candidate position, is further based on the surgical constraint.

Example 8. The device of any one of examples 1-7, wherein the processor is further configured to:
determine a patient position associated with the surgical procedure; and
determine a surgical constraint based on the patient position, wherein the processor being configured to select the candidate position for the second base, from the first candidate position and the second candidate position, is further based on the surgical constraint.

Example 9. A method comprising:
receiving an indication of a plurality of steps of a surgical procedure, wherein one or more steps in the plurality of steps of the surgical procedure involve use of at least one of a first robotic arm attached to a first base, or a second robotic arm attached to a second base;
determining a fixed position of the first base;
determining, based on the plurality of steps of the surgical procedure and the fixed position of the first base, that a first candidate position of the second base is associated with a first number of interactions in which the first robotic arm and the second robotic arm will co-occupy space during the surgical procedure;
determining, based on the plurality of steps of the surgical procedure and the fixed position of the first base, that a second candidate position of the second base is associated with a second number of interactions in which the first robotic arm and the second robotic arm will co-occupy space during the surgical procedure;
selecting a candidate position for the second base, from the first candidate position and the second candidate position, based on the first number of interactions and the second number of interactions; and
generating a control signal configured to indicate the selected candidate position for the second base.

Example 10. The method of example 9, wherein the control signal being configured to indicate the selected candidate position of the second base comprises the control signal being configured to indicate one or more of: the first candidate position, the first number of interactions, the second candidate position, the second number of interactions, and a recommendation for the selected candidate position to be used as a fixed position of the second base.

Example 11. The method of example 9 or 10, wherein the first robotic arm is configured to move a first end effector attached to a distal end of the first robotic arm, and the second robotic arm is configured to move a second end effector attached to a distal end of the second robotic arm, each step in the plurality of steps of the surgical procedure is associated with a surgical space internal to a patient, and wherein the method further comprises identifying a set of candidate positions, comprising the first candidate position and the second candidate position, based on the plurality of steps of the surgical procedure, wherein each candidate position in the set of candidate positions allows the first end effector and the second end effector to access the surgical space at a given step in the plurality of steps of the surgical procedure.

Example 12. The method of any one of examples 9-11, wherein selecting the candidate position, from the first candidate position and the second candidate position, based on the first number of interactions and the second number of interactions comprises:
on a condition that the first number of interactions is less than the second number of interactions, selecting the first candidate position; and
on a condition that the first number of interactions is greater than the second number of interactions, selecting the second candidate position.

Example 13. The method of any one of examples 9-12, wherein one or more steps in the plurality of steps of the surgical procedure involve use of a third robotic arm attached to a third base, and the method further comprises:
determining, based on the plurality of steps of the surgical procedure, the fixed position of the first base, and the selected candidate position, that a third candidate position of the third base is associated with a third number of interactions in which the third robotic arm and at least one of the first robotic arm or the second robotic arm will co-occupy space during the surgical procedure;
determining, based on the plurality of steps of the surgical procedure the fixed position of the first base, and the selected candidate position, that a fourth candidate position of the third base is associated with a fourth number of interactions in which the third robotic arm and at least one of the first robotic arm or the second robotic arm will co-occupy space during the surgical procedure;
selecting a candidate position for the third base, from the third candidate position and the fourth candidate position, based on the third number of interactions and the fourth number of interactions; and
generating a control signal configured to indicate the selected candidate position for the third base.

Example 14. The method of any one of examples 9-13, wherein one or more steps in the plurality of steps of the surgical procedure involve use of a third robotic arm attached to a third base, wherein the method further comprises predicting an effect, caused by the selected candidate position for the second base, on placement of the third base, wherein the control signal is further configured to indicate the effect.

Example 15. The method of any one of examples 9-14, wherein the method further comprises:
receiving user preference information; and
determining a surgical constraint based on the user preference information, wherein selecting the candidate position for the second base, from the first candidate position and the second candidate position, is further based on the surgical constraint.

Example 16. The method of any one of examples 9-15, wherein the method further comprises:
determining a patient position associated with the surgical procedure; and
determining a surgical constraint based on the patient position, wherein selecting the candidate position for the second base, from the first candidate position and the second candidate position, is further based on the surgical constraint.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A device comprising a processor configured to:
receive an indication of a plurality of steps of a surgical procedure, wherein one or more steps in the plurality of steps of the surgical procedure involve use of at least one of a first robotic arm attached to a first base, or a second robotic arm attached to a second base;
determine a fixed position of the first base;
determine, based on the plurality of steps of the surgical procedure and the fixed position of the first base, that a first candidate position of the second base is associated with a first number of interactions in which the first robotic arm and the second robotic arm will co-occupy space during the surgical procedure;
determine, based on the plurality of steps of the surgical procedure and the fixed position of the first base, that a second candidate position of the second base is associated with a second number of interactions in which the first robotic arm and the second robotic arm will co-occupy space during the surgical procedure;
select a candidate position for the second base, from the first candidate position and the second candidate position, based on the first number of interactions and the second number of interactions; and
generate a control signal configured to indicate the selected candidate position for the second base.

2. The device of claim 1, wherein the first robotic arm is configured to move a first end effector attached to a distal end of the first robotic arm, and the second robotic arm is configured to move a second end effector attached to a distal end of the second robotic arm, each step in the plurality of steps of the surgical procedure is associated with a surgical space internal to a patient, and wherein the processor is further configured to identify a set of candidate positions, comprising the first candidate position and the second candidate position, based on the plurality of steps of the surgical procedure, wherein each candidate position in the set of candidate positions allows the first end effector and the second end effector to access the surgical space at a given step in the plurality of steps of the surgical procedure.

3. The device of claim 1 or claim 2, wherein the processor being configured to select the candidate position, from the first candidate position and the second candidate position, based on the first number of interactions and the second number of interactions comprises the processor being configured to:
on a condition that the first number of interactions is less than the second number of interactions, select the first candidate position; and
on a condition that the first number of interactions is greater than the second number of interactions, select the second candidate position.

4. The device of any one of the preceding claims, wherein one or more steps in the plurality of steps of the surgical procedure involve use of a third robotic arm attached to a third base, and the processor is further configured to:
determine, based on the plurality of steps of the surgical procedure, the fixed position of the first base, and the selected candidate position, that a third candidate position of the third base is associated with a third number of interactions in which the third robotic arm and at least one of the first robotic arm or the second robotic arm will co-occupy space during the surgical procedure;
determine, based on the plurality of steps of the surgical procedure the fixed position of the first base, and the selected candidate position, that a fourth candidate position of the third base is associated with a fourth number of interactions in which the third robotic arm and at least one of the first robotic arm or the second robotic arm will co-occupy space during the surgical procedure;
select a candidate position for the third base, from the third candidate position and the fourth candidate position, based on the third number of interactions and the fourth number of interactions; and
generate a control signal configured to indicate the selected candidate position for the third base.

5. The device of any one of the preceding claims, wherein one or more steps in the plurality of steps of the surgical procedure involve use of a third robotic arm attached to a third base, wherein the processor is further configured to predict an effect, caused by the selected candidate position for the second base, on placement of the third base, wherein the control signal is further configured to indicate the effect.

6. The device of any one of the preceding claims, wherein the processor is further configured to:
receive user preference information; and
determine a surgical constraint based on the user preference information, wherein the processor being configured to select the candidate position for the second base, from the first candidate position and the second candidate position, is further based on the surgical constraint.

7. The device of any one of the preceding claims, wherein the processor is further configured to:
determine a patient position associated with the surgical procedure; and
determine a surgical constraint based on the patient position, wherein the processor being configured to select the candidate position for the second base, from the first candidate position and the second candidate position, is further based on the surgical constraint.

8. A method performed by a processor, the method comprising:
receiving an indication of a plurality of steps of a surgical procedure, wherein one or more steps in the plurality of steps of the surgical procedure involve use of at least one of a first robotic arm attached to a first base, or a second robotic arm attached to a second base;
determining a fixed position of the first base;
determining, based on the plurality of steps of the surgical procedure and the fixed position of the first base, that a first candidate position of the second base is associated with a first number of interactions in which the first robotic arm and the second robotic arm will co-occupy space during the surgical procedure;
determining, based on the plurality of steps of the surgical procedure and the fixed position of the first base, that a second candidate position of the second base is associated with a second number of interactions in which the first robotic arm and the second robotic arm will co-occupy space during the surgical procedure;
selecting a candidate position for the second base, from the first candidate position and the second candidate position, based on the first number of interactions and the second number of interactions; and
generating a control signal configured to indicate the selected candidate position for the second base.

9. The device of any one of claims 1 to 7 or the method of claim 8, wherein the control signal being configured to indicate the selected candidate position of the second base comprises the control signal being configured to indicate one or more of: the first candidate position, the first number of interactions, the second candidate position, the second number of interactions, and a recommendation for the selected candidate position to be used as a fixed position of the second base.

10. The method of claim 8 or claim 9, wherein the first robotic arm is configured to move a first end effector attached to a distal end of the first robotic arm, and the second robotic arm is configured to move a second end effector attached to a distal end of the second robotic arm, each step in the plurality of steps of the surgical procedure is associated with a surgical space internal to a patient, and wherein the method further comprises identifying a set of candidate positions, comprising the first candidate position and the second candidate position, based on the plurality of steps of the surgical procedure, wherein each candidate position in the set of candidate positions allows the first end effector and the second end effector to access the surgical space at a given step in the plurality of steps of the surgical procedure.

11. The method of any one of claims 8 to 10, wherein selecting the candidate position, from the first candidate position and the second candidate position, based on the first number of interactions and the second number of interactions comprises:
on a condition that the first number of interactions is less than the second number of interactions, selecting the first candidate position; and
on a condition that the first number of interactions is greater than the second number of interactions, selecting the second candidate position.

12. The method of any one of claims 8 to 11, wherein one or more steps in the plurality of steps of the surgical procedure involve use of a third robotic arm attached to a third base, and the method further comprises:
determining, based on the plurality of steps of the surgical procedure, the fixed position of the first base, and the selected candidate position, that a third candidate position of the third base is associated with a third number of interactions in which the third robotic arm and at least one of the first robotic arm or the second robotic arm will co-occupy space during the surgical procedure;
determining, based on the plurality of steps of the surgical procedure the fixed position of the first base, and the selected candidate position, that a fourth candidate position of the third base is associated with a fourth number of interactions in which the third robotic arm and at least one of the first robotic arm or the second robotic arm will co-occupy space during the surgical procedure;
selecting a candidate position for the third base, from the third candidate position and the fourth candidate position, based on the third number of interactions and the fourth number of interactions; and
generating a control signal configured to indicate the selected candidate position for the third base.

13. The method of any one of claims 8 to 12, wherein one or more steps in the plurality of steps of the surgical procedure involve use of a third robotic arm attached to a third base, wherein the method further comprises predicting an effect, caused by the selected candidate position for the second base, on placement of the third base, wherein the control signal is further configured to indicate the effect.

14. The method of any one of claims 8 to 13, wherein the method further comprises:
receiving user preference information; and
determining a surgical constraint based on the user preference information, wherein selecting the candidate position for the second base, from the first candidate position and the second candidate position, is further based on the surgical constraint.

15. The method of any one of claims 8 to 14, wherein the method further comprises:
determining a patient position associated with the surgical procedure; and
determining a surgical constraint based on the patient position, wherein selecting the candidate position for the second base, from the first candidate position and the second candidate position, is further based on the surgical constraint.

16. A computer-readable medium comprising instructions which, when executed by a processor, cause the processor to carry out the method of any one of claims 8 to 15.
